(19) Europäisches Patentamt
European Patent Office
Office européen des brevets

(11) EP 0 638 317 B1

# (12) EUROPÄISCHE PATENTSCHRIFT

(45) Veröffentlichungstag und Bekanntmachung des Hinweises auf die Patenterteilung:
**15.03.2000 Patentblatt 2000/11**

(51) Int. Cl.[7]: **A61K 45/06**, A61K 31/70, A61K 31/365

(21) Anmeldenummer: **94111436.5**

(22) Anmeldetag: **22.07.1994**

(54) **Pharmazeutisches Präparat enthaltend Glucosidase- und/oder Amylasehemmer und einen Lipasehemmer**

Pharmaceutical composition comprising glucosidase- and/or amylaseinhibitors, and a lipase inhibiting agent

Composition pharmaceutique contenante des inhibiteurs du glucosidase et/ou de l'amylase, et un inhibiteur de lipase

(84) Benannte Vertragsstaaten:
**AT BE CH DE DK ES FR GB GR IE IT LI LU MC NL PT SE**

(30) Priorität: **05.08.1993 CH 233993**

(43) Veröffentlichungstag der Anmeldung:
**15.02.1995 Patentblatt 1995/07**

(73) Patentinhaber:
**F. HOFFMANN-LA ROCHE AG
4002 Basel (CH)**

(72) Erfinder:
• **Bremer, Klaus-Dieter
CH-4123 Allschwil (CH)**
• **Sawlewicz, Pavel
CH-4055 Basel (CH)**

(74) Vertreter: **Mahé, Jean et al
F.Hoffmann-La Roche AG
Patent Department (PLP),
124 Grenzacherstrasse
4070 Basel (CH)**

(56) Entgegenhaltungen:
**EP-A- 0 129 748        EP-A- 0 364 696
WO-A-89/11295        WO-A-92/05788
WO-A-93/07872        US-A- 4 189 438
US-A- 5 240 962**

• **M. NEGWER "Organic-chemical drugs and their synonyms", 6. Auflage, Vol. II, 1987, AKADEMIE-VERLAG BERLIN, Seiten 1310,1311,1547,1652 * Seiten 1310,1311, Nr. 7359; Seite 1547, Nr. 8406; Seite 1652, Nr. 9034 ***
• **B.W. BYCROFT "Dictionary of Antibiotics and Related Substances", 1988, CHAMPMAN AND HALL, London, New York Seiten 15,298,315 445,698 * Seite 15, Abschnitte "Adiposin I", Adiposin II; Seite 298, Abschnitte "Ebelactone A", "Ebelactone B"; Seite 315, Abschnitt "Esterasin"; Seite 445, Abschnitt "Lipstatin"; Seite 698, Abschnitte "Trestatin A", "Trestatin B", "Trestatin C" ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.7/3.6

**Beschreibung**

Die vorliegende Erfindung betrifft pharmazeutische Präparate, enthaltend als Wirkstoffe einen Glucosidase- und/oder Amylasehemmer und einen Lipasehemmer und übliche pharmazeutische Trägerstoffe.

Es wurde gefunden, dass man solche Präparate zur Behandlung der Fettsucht verwenden kann.

Die Erfindung betrifft somit auch die Verwendung eines Glucosidase- und/oder Amylasehemmers in Kombination mit einem Lipasehemmer zur Herstellung eines pharmazeutischen Präparates zur Behandlung von Fettsucht.

Beispiele von erfindungsgemäss verwendbaren Glucosidase- und/oder Amylasehemmer sind Acarbose, Adiposine, Voglibose (AO-128), Miglitol (Bay-m-1099), Emiglitate (Bay-o-1248), MDL-25637, Camiglibose (MDL-73945), Tendamistate, AI-3688, Trestatin, Pradimicin-Q und Salbostatin.

Beispiele von Lipasehemmer sind Tetrahydrolipstatin, Lipstatin, FL-386, WAY-121898, Bay-N-3176, Valilactone, Esterastin, Ebelactone A, Ebelactone B und RHC 80267.

Als Lipasehemmer kann man auch die bei der fermentativen Herstellung von Lipasehemmern, wie Lipstatin oder Esterastin, entstehenden Biomassen oder Gärkuchen verwenden. Letztere sind z.B. in der EP-A 129 748 und dem USP 4 189 438 beschrieben.

Von Glucosidase- und/oder Amylasehemmer, wie Acarbose, ist bekannt, dass sie die Verdauung der Kohlenhydrate verlangsamen.

Bekannt ist ferner, dass Lipasehemmer, wie Tetrahydrolipstatin (Orlistat), zu einer partiellen Hemmung der Lipase im Darm führen.

Bei Einzeltherapie führen jedoch im allgemeinen die Lipasehemmer selbst bei Kombination mit einer Reduktionsdiät nur zu einem mässigen, und die Glucosidase- und/oder Amylasehemmer zu praktisch keinem Gewichtsverlust. Ueberraschenderweise wurde nun festgestellt, dass eine kombinierte Anwendung eines Glucosidase- und/oder Amylasehemmers und eines Lipasehemmers zu einem wesentlich höheren Gewichtsverlust als bei der Einzeltherapie führt. Dies wurde im folgenden Test nachgewiesen:

Der Test wurde in zwei Versuchsperioden an zwei Probanden (A und B) durchgeführt. In einem 7-tägigen Vorversuch, in dem die Probanden keine Medikation einnahmen, wurden mittlere Tageskalorienmengen von 2560 bzw. 1850 Kcal für A bzw. B ermittelt. Im anschliessenden 14-tägigen Hauptversuch erhielten die Probanden bei jeder Mahlzeit 120 mg Orlistat und 100 mg Acarbose. Dabei wurde keine spezielle Diät eingehalten und die physischen Aktivitäten auf ein Minimum reduziert. So wie im Vorversuch wurden auch hier die mittleren Tageskalorienmengen von 2185 bzw. 2050 Kcal für A bzw. B ermittelt. Aus der nachstehenden Tabelle ist der Gewichtsverlust beider Probanden ersichtlich.

| Versuchstag | Körpergewicht (kg) | |
|---|---|---|
| | A | B |
| 1 | 74,3 | 88,7 |
| 2 | 74,1 | 88,5 |
| 3 | 74,6 | 89,1 |
| 4 | 73,9 | 88,0 |
| 5 | 73,5 | 88,4 |
| 6 | 73,3 | 88,2 |
| 7 | 73,0 | 87,7 |
| 8 | 73,6 | 87,8 |
| 9 | 73,3 | 87,7 |
| 10 | 73,1 | 87,4 |
| 11 | 72,8 | 87,2 |
| 12 | 72,4 | 87,5 |
| 13 | 72,4 | 87,2 |
| 14 | 72,2 | 86,4 |
| 15 | 71,9 | 86,1 |

(fortgesetzt)

| Versuchstag | Körpergewicht (kg) | |
|---|---|---|
| | **A** | **B** |
| Gewichtsverlust | 2,4 | 2,6 |

Im Vergleich hierzu betrug der Gewichtsverlust von Patienten bei Einzeltherapie mit Orlistat (3 x 120 mg/Tag) in einem Plazebo-kontrollierten 12-Wochen-Versuch im Mittel 1,8 kg (d.h. 0,3 kg/14 Tage) (Int. J. Obesity 1992; 16 (Suppl. 1): 16, Abstr. 063).

Erfindungsgemäss kann ein Glucosidase- und/oder Amylasehemmer in Form pharmazeutischer Präparate, die auch einen Lipasehemmer enthalten, oder als ad hoc-Kombination mit Lipasehemmer enthaltenden Präparaten angewandt werden.

Bevorzugt ist die Verwendung von Acarbose und Orlistat.

Zur Behandlung der Fettsucht werden die Wirkstoffe oral verabreicht.

Dabei können sie in Dosierungen von etwa 0,003 mg bis etwa 20 mg, vorzugsweise 0,015 mg bis 10 mg Glucosidase- und/oder Amylasehemmer und von etwa 0,15 mg bis 20 mg, vorzugsweise von 0,5 mg bis 10 mg Lipasehemmer pro kg Körpergewicht täglich verabreicht werden.

Die erfindungsgemässen oral zu verabreichenden Präparate können in Form von Tabletten, Kapseln, Lösungen oder Emulsionen vorliegen.

Feste Darreichungsformen, wie Tabletten und Kapseln enthalten pro Dosiseinheit zweckmässigerweise etwa 0,2 mg bis etwa 100 mg Glucosidase-und/oder Amylasehemmer und 10 mg bis 200 mg Lipasehemmer.

Ausser zur Behandlung der Fettsucht können die erfindungsgemässen Präparate bzw. Wirkstoffkombination zur Behandlung und Prävention von Krankheiten, welche häufig mit Uebergewicht vergesellschaftet auftreten, verwendet werden, wie Diabetes, Hypertension, Hyperlipidämie und Insulinresistenz-Syndrom.

Bei allen diesen Indikationen können die Wirkstoffe in den oben angegebenen Dosierungsbereichen angewandt werden, wobei die individuelle Dosierung von der Art der zu behandelnden Erkrankung sowie vom Alter und Zustand des Patienten abhängig und im Rahmen des ärztlichen Fachwissens bestimmt werden kann. Die Erfindung wird durch die nachstehenden Beispiele weiter erläutert.

In an sich bekannter Weise werden pharmazeutische Präparat folgender Zusammensetzung hergestellt:

| Beispiel A Weichgelatinekapseln: | |
|---|---|
| | Menge pro Kapsel |
| Orlistat | 60 mg |
| Mittelkettiges Triglycerid | 450 μl |
| Acarbose | 50 mg |

| Beispiel B Hartgelatinekapseln: | |
|---|---|
| Acarbose | 25,0 mg |
| Orlistat | 30,0 mg |
| Milchzucker krist. | 37,0 mg |
| Mikrokristalline Cellulose | 20,0 mg |
| Polyvinylpolypyrrolidon | 8,5 mg |

(fortgesetzt)

| Beispiel B Hartgelatinekapseln: | |
|---|---|
| Natrium-Carboxymethyl-stärke | 8,5 mg |
| Talk | 4,5 mg |
| Magnesiumstearat | 1,5 mg |
| Kapselfüllgewicht | 135,0 mg |

| Beispiel C Tablette: | |
|---|---|
| Acarbose | 25,0 mg |
| Orlistat | 30,0 mg |
| Milchzucker wasserfrei | 118,8 |
| Mikrokristalline Cellulose | 30,0 |
| Polyvinylpolypyrrolidon | 10,0 |
| Carboxymethylcellulose | 10,0 |
| Magnesiumstearat | 1,2 |
| Tablettengewicht | 225,0 mg |

| Beispiel D Tablette mit kontrollierter Wirkstoffabgabe und erhöhter Verweilzeit im Magen: | |
|---|---|
| Acarbose | 50,0 mg |
| Orlistat | 60,0 mg |
| Milchzucker pulv. | 70,0 mg |
| Hydroxypropylmethylcellulose | 52,5 mg |
| Polyvinylpolypyrrolidon | 7,5 mg |
| Talk | 8,0 mg |
| Magnesiumstearat | 1,0 mg |
| kolloidale Kieselsäure | 1,0 mg |
| Kerngewicht | 250,0 mg |
| Hydroxypropylmethylcellulose | 2,5 mg |
| Talk | 1,25 mg |
| Titandioxid | 1,25 mg |
| Gewicht des Filmüberzugs | 5,0 mg |

| Beispiel E Rekonstituierbares Pulver: | | |
|---|---|---|
| Acarbose | | 100,0 mg |
| Orlistat | | 120,0 mg |
| Aethylvanillin | | 10,0 mg |
| Aspartam | | 30,0 mg |
| Sprühmagermilchpulver | | 4740,0 mg |
| | Total | 5'000,0 mg |

**Patentansprüche**

1. Pharmazeutisches Präparat, enthaltend als Wirkstoffe einen Glucosidase- und/oder Amylasehemmer und einen Lipasehemmer und übliche pharmazeutische Trägerstoffe.

2. Präparat nach Anspruch 1, worin der Glucosidase- und/oder Amylasehemmer Acarbose, Adiposine, Voglibose (AO-128), Miglitol (Bay-m-1099), Emiglitate (Bay-o-1248), MDL-25637, Camiglibose (MDL-73945), Tendamistate, AI-3688, Trestatin, Pradimicin-Q oder Salbostatin ist.

3. Präparat nach Anspruch 1, worin der Lipasehemmer, Tetrahydrolipstatin, Lipstatin, FL-386, WAY-121898, Bay-N-3176, Valilactone, Esterastin, Ebelactone A, Ebelactone B oder RHC 80267 ist.

4. Präparat nach Anspruch 1, 2 oder 3, enthaltend Acarbose und Tetrahydrolipstatin als Wirkstoffe.

5. Erzeugnisse enthaltend einen Glucosidase- und/oder Amylasehemmer und einen Lipasehemmer als Kombinationspräparat zur gleichzeitigen, getrennten oder zeitlich abgestuften Anwendung bei der Behandlung der Fettsucht.

6. Erzeugnisse gemäss Anspruch 5 enthaltend einen der in Anspruch 2 bzw. 3 aufgezählten Glucosidase- und/oder Amylasehemmer bzw. Lipasehemmer, insbesondere Acarbose und Tetrahydrolipstatin.

7. Verwendung eines Glucosidase- und/oder Amylasehemmers in Kombination mit einem Lipasehemmer zur Herstellung eines pharmazeutischen Präparates zur Behandlung von Fettsucht.

8. Verwendung von Acarbose in Kombination mit Tetrahydrolipstatin gemäss Anspruch 7.

**Claims**

1. A pharmaceutical preparation containing a glucosidase and/or amylase inhibitor and a lipase inhibitor as the active substances and usual pharmaceutical carriers.

2. A preparation according to claim 1, wherein the glucosidase and/or amylase inhibitor is acarbose, adiposine, voglibose (AO-128), miglitol (Bay-m-1099), emiglitate (Bay-o-1248), MDL-25637, camiglibose (MDL-73945), tendamistate, AI-3688, trestatin, pradimicin-Q or salbostatin.

3. A preparation according to claim 1, wherein the lipase inhibitor is tetrahydrolipstatin, lipstatin, FL-386, WAY-121898, Bay-N-3176, valilactone, esterastin, ebelactone A, ebelactone B or RHC 80267.

4. A preparation according to claim 1, 2 or 3 containing acarbose and tetrahydrolipstatin as the active substances.

5. A product containing a glucosidase and/or amylase inhibitor and a lipase inhibitor as a combination preparation for the simultaneous, separate or chronologically spaced use in the treatment of obesity.

6. A product according to claim 5 containing a glucosidase and/or amylase inhibitor set forth in claim 2 and a lipase inhibitor set forth in claim 3, especially acarbose and tetrahydrolipstatin.

7. The use of a glucosidase and/or amylase inhibitor in combination with a lipase inhibitor for the production of a pharmaceutical preparation for the treatment of obesity.

8. The use of acarbose in combination with tetrahydrolipstatin in accordance with claim 7.

**Revendications**

1. Préparation pharmaceutique contenant en tant que principes actifs un inhibiteur de glucosidase et/ou d'amylase et un inhibiteur de lipase, et des excipients pharmaceutiques usuels.

2. Préparation selon la revendication 1, dans laquelle l'inhibiteur de glucosidase et/ou d'amylase est l'acarbose, l'adiposine, la voglibose (AO-128), le miglitol (Bay-m-1099), l'émiglitate (Bay-O-1248), le MDL-25637, le camiglibose (MDL-73945), le tendamistate, l'AI-3688, la trestatine, la pradimicine-Q ou la salbostatine.

3. Préparation selon la revendication 1, dans laquelle l'inhibiteur de lipase est la tétrahydrolipstatine, la lipstatine, le FL-386, le WAY-121898, le Bay-N-3176, la valilactone, l'estérastine, l'ébélactone A, l'ébélactone B ou le RHC 80267.

4. Préparation selon la revendication 1, 2 ou 3, qui contient en tant que principes actifs de l'acarbose et de la tétrahydrolipstatine.

5. Produits contenant un inhibiteur de glucosidase et/ou d'amylase et un inhibiteur de lipase en tant que préparation en association pour utilisation simultanée, séparée ou étagée dans le temps lors du traitement de l'adipose.

6. Produits selon la revendication 5, contenant un inhibiteur de glucosidase et/ou d'amylase ou un inhibiteur de lipase mentionnés dans la revendication 2 ou 3, en particulier l'acarbose et la tétrahydrolipstatine.

7. Utilisation d'un inhibiteur de glucosidase et/ou d'amylase en association avec un inhibiteur de lipase pour préparer une préparation pharmaceutique destinée au traitement de l'adipose.

8. Utilisation d'acarbose en association avec la tétrahydrolipstatine selon la revendication 7.